(19) Europäisches Patentamt
European Patent Office
Office européen des brevets

(11) Publication number: 0 003 258
B1

# (12) EUROPEAN PATENT SPECIFICATION

(45) Date of publication of patent specification: 13.01.82

(21) Application number: 78300802.2

(22) Date of filing: 13.12.78

(51) Int. Cl.³: C 07 D 209/48,
C 07 D 207/46,
C 07 D 275/06,
C 07 D 487/10
//(C07D487/10, 209/00,
209/00)

## (54) Preparation of N-chloroimides and a novel N-chloroimide.

(30) Priority: 19.12.77 US 861734
19.12.77 US 861582

(43) Date of publication of application:
08.08.79 Bulletin 79/16

(45) Publication of the grant of the European patent:
13.01.82 Bulletin 82/2

(84) Designated Contracting States:
DE GB IT NL SE

(56) References cited:
DE - A - 2 721 738

(73) Proprietor: ELI LILLY AND COMPANY
307, East McCarty Street
Indianapolis Indiana 46206 (US)

(72) Inventor: Wright, Ian Glaisby
300, Meander Way
Greenwood Indiana 46142 (US)
Inventor: Chou, Ta-Sen
745, Canyon Road
Indianapolis Indiana 46217 (US)

(74) Representative: McVey, Kenneth William Henry
et al,
Lilly House 13 Hanover Square
London W1R 0PA (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 003 258 B1

Courier Press, Leamington Spa, England.

# Preparation of N-chloroimides and a novel N-chloroimide

The invention relates to the chlorination of imides to prepare N-chloroimides and to a novel N-chloroimide.

The principal prior art processes for preparing N-chloroimides customarily have involved the use of an aqueous system. In general, such prior art processes for preparing N-chloroimides can be classified as follows:

(1) Chlorination of the corresponding imide using an inorganic hypochlorite in a mixture of acetic acid and water;

(2) Chlorination by passing chlorine into an aqueous solution comprising equivalent amounts of the corresponding imide and a strong base, e.g., sodium hydroxide or potassium hydroxide;

(3) Chlorination of the corresponding imide using *t*-butyl hypochlorite in a mixture of *t*-butyl alcohol and water.

Of the above general methods, only method (2) prescribes the use of chlorine itself in the production of the N-chloroimide. However, due to the fact of the aqueous system, this method has been found to have serious drawbacks. First, chlorine is only very slightly soluble in water. Secondly, and more importantly, it is known that an imide, when present in an alkaline aqueous medium such as would result from potassium or sodium hydroxide and water, undergoes rapid hydrolysis.

Even more importantly, it has been established [Hurwitz, "Degradation of N-Chlorosuccinimide in Aqueous Solution", *Diss. Abst.*, B, *28* (3), 971 (1967)] that an N-chloroimide product, when present in an aqueous alkaline medium, such as would be the case under the conditions of chlorination provided by method (2) above, degrades with possible formation of the highly explosive and toxic gas, nitrogen trichloride.

Non-aqueous processes for preparing N-chloro compounds have been few. A process for preparing N-halo-*t*-alkyl cyanamides is described in U.S. Patent No. 2,686,203. This process treats a *t*-alkyl cyanamide with molecular chlorine in an inert solvent and in the presence of a molar equivalent of a halogen acid acceptor, typically pyridine.

Recently, a method for preparing N-chlorophthalimide under substantially non-aqueous reaction conditions was discovered. The aforementioned deficiencies of an aqueous alkaline medium thus were avoided by this method which involves contacting an alkali metal salt of phthalimide with chlorine under non-aqueous conditions in the presence of a halogenated aliphatic hydrocarbon and at a temperature of from about −10°C. to about +40°C. Belgian Patent 853,686.

This invention provides an even more advantageous process for preparing N-chloroimides, which comprises contacting the corresponding imide with molecular chlorine under substantially anhydrous conditions in the presence of (1) an epoxy compound in an amount representing at least about one epoxy moiety per each imide moiety and (2) at least a catalytic amount of a tertiary amine.

More particularly, the invention comprises contacting an imide containing one or more of the moieties.

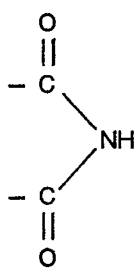

or

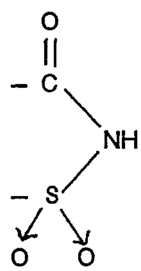

with molecular chlorine as described above.

In more preferred embodiments, the invention comprises contacting with molecular chlorine, as described above, the imides phthalimide, succinimide, saccharin, glutarimide, 3,3-dimethylglutarimide, maleimide, $\Delta^4$-tetrahydrophthalimide, 3,4,5,6-tetrachlorophthalimide, pyromellitic diimide, benzophenone tetracarboxylic diimide, naphthalene 1,4,5,8-tetracarboxylic diimide, chlorendic imide, carbonyl salicylamide, diphenimide, tetrahydrofuran tetracarboxylic imide, 1,8-naphthalimide, or bicyclo[2.2.2]oct-7-ene-2,3,5,6-tetracarboxylic-2,3,5,6-diimide, to prepare the corresponding N-chloroimides.

The present invention is advantageous in that it avoids the decomposition likely to result from the aqueous chlorination processes, and is carried out on the imide itself, rather than on a salt thereof.

N-chloroimides, the products of the process of this invention, have long been recognized as reagents useful in reactions requiring a source of positive chlorine. Examples of such reactions are, for example, oxidation of alcohols, sulfides, amines, and imines; chlorination of amines, reactive aromatic systems, carbonyl compounds having $\alpha$-hydrogens, and the like. Publications which describe such reactions include R. Filler, *Chem. Revs. 63,* 21 (1963); and R. Stroh, "Methoden der Organischen

Chemie" (Houben-Weil), Vol. 5, Part 3, pp. 760—762, 796 *et seq.,* Georg Thieme Verlag, Stuttgart (1962).

Recently, it has been discovered that a penicillin sulfoxide can be converted to the corresponding 3-methylenecepham sulfoxide by the following sequence:

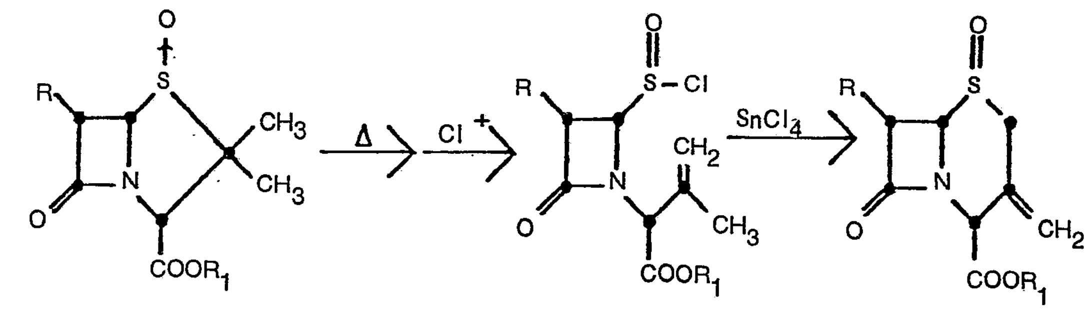


This reaction is reported in Kukolja *et al., Journal of the American Chemical Society, 98,* 5040 (1976) and is further elaborated in Belgian Patent No. 837,040. The first step contemplates thermal generation of the sulfenic acid which then is trapped by oxidation to the sulfinyl chloride. The sulfinyl chloride is cyclized to the 3-exomethylenecepham sulfoxide using a Lewis acid reagent, in particular, stannic chloride. The first step, formation of the sulfinyl chloride, requires use of a positive chlorine reagent, and, in particular, an N-chloroimide. Particularly preferred N-chloroimides in accordance with Belgian Patent No. 837,040 include N-chlorophthalimide, N-chlorosuccinimide, and N-chloroglutarimide.

In carrying out the above penicillin sulfoxide reaction, it is desirable to employ an N-chloroimide having certain properties. First, it is desirable to use an N-chloroimide having a level of reactivity which is neither too great nor too small, that is, a level which provides reaction at readily available conditions while being sufficiently unreactive and stable to provide ease of handling and use in the intended reaction. Secondly, it is desirable to employ an N-chloroimide which will produce, as byproduct, a corresponding imide which is sufficiently insoluble in the reaction system to permit ready removal from the reaction medium and, thus, easy isolation of the desired product.

These properties are provided by the novel N-chloroimide, N,N'-dichlorobicyclo[2.2.2]oct-7-ene-2,3,5,6-tetracarboxylic-2,3,5,6-diimide of the formula

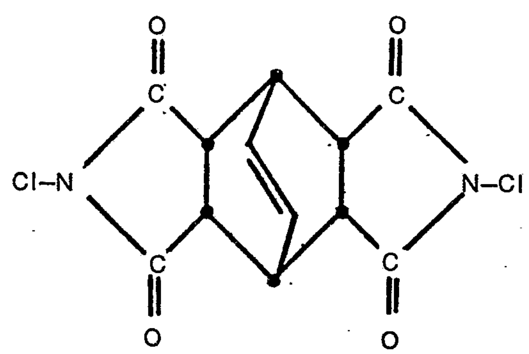

(I)

As indicated, the above compound is highly suitable for use as a positive chlorine source in the reaction reported by Kukolja *et al.* since (1) it is both sufficiently stable to permit ready use and sufficiently reactive to provide a ready source of positive chlorine, and (2) it gives rise to a highly insoluble diimide byproduct which can be conveniently removed from the reaction medium. The stability of the compound is indicated by the fact that it melts with decomposition only after being heated to a temperature of 266°C. Furthermore, it remains unaffected after extended periods, for example, 16 hours, of toluene reflux. Other more reactive N-chloroimides are at least partially destroyed by reaction with the solvent when the mixture is maintained for extended periods at elevated temperature. For example, N-chloro-succinimide is reported to react with toluene (110°C., 16 hours), to produce both ring- and $\alpha$-substitution. [C. Yavoslavsky and E. Katchalski, *Tetrahedron Letters, 51,* 5173 (1972)].

Moreover, the compound of formula I is sufficiently soluble in organic solvents which are commonly employed in carrying out positive chlorine reactions to permit such reaction to occur. For example, its solubility in boiling toluene is approximately 3 g. per liter. Conversely, its corresponding diimide, the byproduct from a positive chlorine reaction, is almost entirely insoluble and thus is readily removable from the reaction mixture. The solubility of the diimide in boiling toluene is less than 25 mg. per liter. Moreover, the diimide byproduct is highly stable and crystalline, having a melting point in excess of 410°C.

The compound of formula I is most advantageously prepared by the use of the novel process which has been described. It may also be made, however, by the common processes of the prior art by N-chlorinating bicyclo[2.2.2]oct-7-ene-2,3,5,6-tetracarboxylic-2,3,5,6-diimide.

A useful prior art process for such N-chlorination is illustrated in Example 16 below, where the diimide is N-chlorinated with molecular chlorine in acetic acid in the presence of an acetate. The reaction occurs efficiently at temperatures from 0°C. to about 80°C.

The chlorination which prepares the compound of formula I may also be carried out with other prior art N-chlorinating agents, of which hypochlorites are best known and most often used. In particular, tertiary butyl hypochlorite may be used, and alkali metal hypochlorites such as sodium and calcium hypochlorites have been commonly employed in the prior art for N-chlorination. Further, still other well known prior art N-chlorination procedures can be used to prepare the compound of formula I. For example, 1-chlorobenzotriazole is an adequate reagent, and the chloroisocyanuric acids are also used as N-chlorinating agents.

Exemplary publications concerning the preparation of N-chloroimides include Filler and Stroh, cited above, and Oelschlaeger et al., *Pharm. Acta. Helv.* 47, 1—6 (1972); Matsushim et al., *Biochem. Biophys. Acta. 271,* 243—51 (1972); and Cinquini et al., *Synthesis* 1972, 259—60.

As is explained in prior art such as the above, the commonly used N-chlorinating agents are effective when used in solvents such as the chlorinated aliphatic and aromatic solvents as discussed below, and also in aqueous reaction mixtures. In general, reaction temperatures from 0°C. to 50°C. are effective and are commonly used.

As indicated hereinabove, this invention provides a process for preparing N-chloroimides. The process of this invention involves the interaction of molecular chlorine with the imide corresponding to the intended N-chloroimide product under substantially anhydrous conditions.

The reaction defined by the process of this invention is equimolar in the sense that one mole of chlorine is consumed for each mole of available imide moiety. Therefore, it is highly preferred that at least one mole of chlorine is present per mole of imide moiety. Even more preferably, about a 10% molar excess of chlorine is brought into contact with the imide.

The temperature at which the reaction is carried out is not critical, but generally ranges from about −10°C. to about +50°C. and, preferably, from about −5°C. to about +25°C. The reaction generally is completed after a period of from about 1 hour to about 24 hours, and, preferably, is carried out over a period of from about 3 to 15 hours.

A further feature of the process of this invention is that it can be and is carried out under substantially anhydrous conditions. It is not intended by the term "substantially anhydrous conditions" to mean the total absence of water from the reaction system; instead, this term prescribes the exercise of reasonable precautions to ensure its preclusion, including the avoidance of any deliberate addition of water to the reaction medium prior to or during the time in which the reaction is being effected. Amounts of water which are customarily present in such commercial solvents and reactants as may be employed in the process of this invention need not first be removed in order to comply with the "substantially non-aqueous" requirement. In general, amounts of water up to about 0.2% by weight of the reaction mixture are not harmful.

Although it is not an essential feature of the process of this invention, the reaction can be carried out in the presence of an inert organic solvent. By the term "solvent" is intended that medium which partially or completely solubilizes the imide starting material. The term "inert" defines a solvent which generally does not react with the reactants, principally, with the chlorine, under the conditions of the process. Typical solvents are halogenated aromatic and aliphatic hydrocarbons. Examples of halogenated aromatic hydrocarbons are chlorobenzene, 1,2-dichlorobenzene, 1,4-dichlorobenzene, bromobenzene, and the like. Examples of halogenated aliphatic hydrocarbons are methylene chloride, chloroform, 1,1,2-trichloroethane, 1,2-dichloroethane, 1,1-dichloroethane, 1,1,1-trichloroethane, and the like. Of the above, the halogenated aliphatic hydrocarbons are preferred, and, of these, the preferred solvent is methylene chloride.

The imide starting material employed in the process of this invention is defined broadly as a diacylamine compound and specifically as a compound containing one or more of either or both of the following moieties:

II 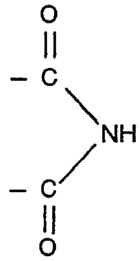 or 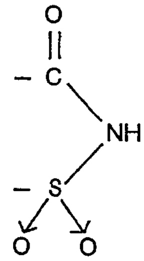 III

Preferably, these imides do not contain additional functional groups which would be reactive under the conditions of this invention. Typical such functional groups, the presence of which preferably is avoided,

are ether, epoxy, hydroxy, amino, sulfide, sulfoxide, carboxyl, and active methylene as well as other such groups having at least one acidic hydrogen.

Illustrations of specific imides which are particularly suitable for the process of this invention are phthalimide, succinimide, saccharin, glutarimide, 3,3-dimethylglutarimide, maleimide, $\Delta^4$-tetrahydrophthalimide, 3,4,5,6-tetrachlorophthalimide, pyromellitic diimide, benzophenone tetracarboxylic diimide

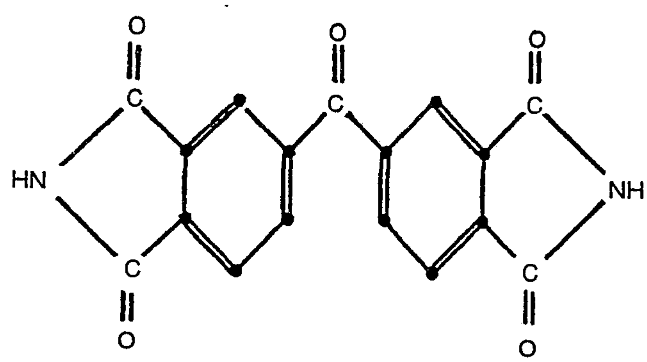

naphthalene 1,4,5,8-tetracarboxylic diimide

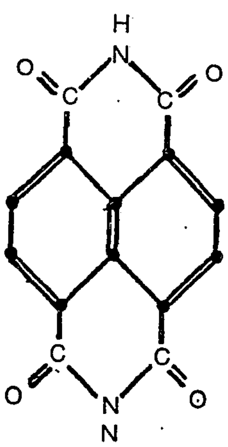

chlorendic imide

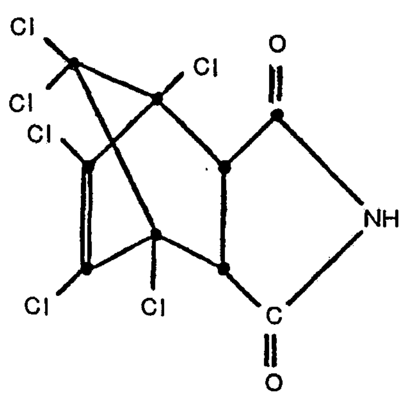

carbonyl salicylimide

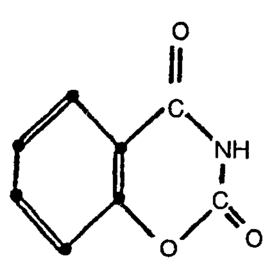

diphenimide

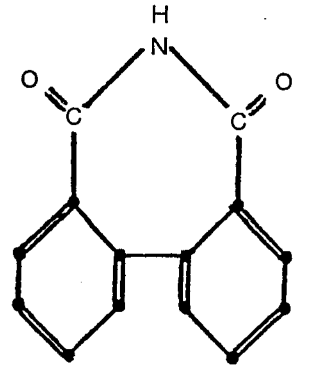

tetrahydrofuran tetracarboxylic diimide

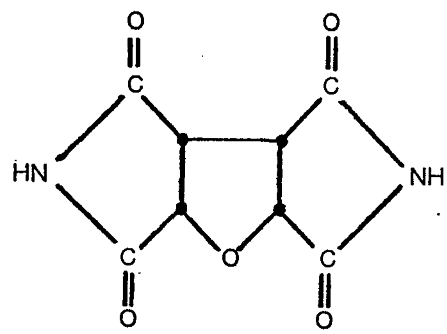

1,8-naphthalimide

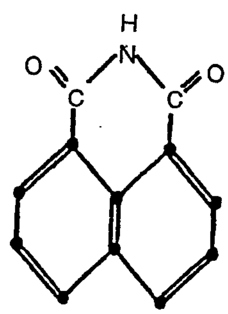

and

bicyclo[2.2.2]oct-7-ene-2,3,5,6-tetracarboxylic-2,3,5,6-diimide.

The foregoing is intended to be illustrative only and is indicative of the wide variety of imides, including polyimides, which are suitable for use in the process of this invention. Particularly preferred imides for use in the process of this invention are phthalimide, succinimide, saccharin, and bicyclo[2.2.2]oct-7-ene-2,3,5,6-tetracarboxylic-2,3,5,6-diimide. Of the above, the most preferred imides are phthalimide and bicyclo[2.2.2]oct-7-ene-2,3,5,6-tetracarboxylic-2,3,5,6-diimide.

In addition to the principal reactants, that is, molecular chlorine and the selected imide, the process of this invention contemplates the use of a catalyst and an acid scavenger. The catalyst and scavenger are believed to work in combination to effect removal of the hydrogen chloride by-product which forms in the process of this invention during reaction of the molecular chlorine with the imide. It is believed that the scavenger serves as the ultimate repository for the hydrogen chloride while the catalyst serves as a transfer agent by which the generated hydrogen chloride by-product is made available for pick-up by the scavenger.

In the context of this invention, the scavenger is an epoxy compound. The epoxy compound is present in an amount at least equivalent to the amount of imide starting material. As used in this context, the term "equivalent" means the presence of at least one epoxy moïety per imide moiety. A large excess of the epoxy compound can be employed without detriment. However, for the sake of convenience and economy, it is preferred that the epoxy compound be present in an amount of from about 1.1 to about 5 equivalents per equivalent of the imide starting material. It is further preferred to employ an epoxy compound having a terminal epoxide group, that is, one containing the moiety

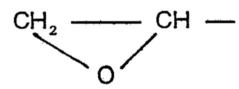

Typical preferred epoxy compounds are ethylene oxide, propylene oxide, 1,2-epoxybutane, butadiene diepoxide, 1,2-epoxy-3-phenoxypropane, 1,4-butanediol diglycidyl ether, 1,2,7,8-diepoxyoctane, and the like. The most preferred epoxy compounds are propylene oxide and 1,2-epoxybutane.

In combination with the scavenger, a catalyst is employed. The catalyst is present in a small but significant quantity, generally from about 0.01 to about 0.04 mole equivalents per equivalent of the imide moiety present in the imide starting material. A larger amount can be employed without detriment. The catalyst which is employed in the process of this invention is a tertiary amine having properties which make it suitable to serve as a catalytic transfer agent. By "catalytic transfer agent" is intended a tertiary amine which achieves transfer of the hydrogen chloride by-produce from the site of the protonated N-chloroimide intermediate to the scavenger where it is permitted to react with the scavenger and thereby be removed from the reaction medium. The tertiary amine which is employed has characteristics which permit formation of its hydrochloride salt by deprotonation of the protonated N-chloroimide intermediate. The resulting hydrochloride salt is sufficiently soluble in the reaction medium to permit its transport to the scavenger and sufficiently unstable to permit reaction with the scavenger. Thus, the tertiary amine catalyst has the following characteristics:

(1) ability to generate its hydrochloride salt by reaction with the protonated N-chloroimide intermediate; and

(2) generation of a hydrochloride salt, the properties of which include:

(a) at least partial solubility in the reaction medium, and

(b) sufficient instability to react with the scavenger with removal of the hydrogen chloride and regeneration of the free tertiary amine.

Typical tertiary amines which are suitable for use in the process of this invention are 1,5-diazabicyclo[4.3.0]non-5-ene (DBN), 1,5-diazabicyclo[5.4.0]undec-5-ene (DBU), pyridine, 1,8-bis(dimethylamino)naphthalene, 4-dimethylaminopyridine, N,N-dimethylbenzylamine, diisopropylethylamine, quinoline, 2,4,6-trimethylpyridine, pyrimidine, pyrazine, and the like. In general, pyridine or structural derivatives of pyridine, including quinolines, are preferred for use as catalysts in the process of this invention. Of high preference are pyridine and, especially, quinoline.

An example of a typical preparation of an N-chloroimide in accordance with the process of this invention is as follows:

Phthalimide (0.6 mole) is slurried in methylene chloride (about 1.7 liters or less), and the mixture is chilled to about 0—5°C. 1,2-Epoxybutane (3.0 moles) and quinoline (0.006 mole) are added. Sufficient chlorine (about 0.66 mole), either as a gas or a liquid, is added by introducing it below the surface of the slurry by means of an inlet tube. The cooling bath then is removed, and the mixture is allowed to warm to a temperature not in excess of about 25°C. After about 1—3 hours, the phthalimide starting material will be gone or nearly gone, and the mixture will have slowly cleared to a yellow solution. A partial vacuum then is applied to the mixture for a few minutes to remove excess chlorine, and amylene (2-methyl-2-butene) is added to take up the remaining chlorine leaving a pale straw-colored reaction mixture. Some exothermicity may be noted as the amylene reacts with the excess chlorine. The excess 1,2-epoxybutane and methylene chloride are removed *in vacuo* at a temperature not in excess of about 25—30°C. The N-chlorophthalimide product crystallizes out of solution as the solvent is removed. The volume of the mixture is reduced to approximately 130—150 ml. and comprises mainly product and butylene chlorohydrin. The mixture is chilled in ice for about one hour and filtered, and the collected product is washed with a small volume of cold toluene or petroleum ether. The product is vacuum dried at 25°C. for about 18 hours to obtain the product as colorless to off-white granular crystals in an amount representing a 92—96% yield.

## Example 1

To 150 ml. of methylene chloride were added 7.35 g. (0.05 mole) of phthalimide, 21.5 ml. (5 equiv.) of 1,2-epoxybutane, and 0.061 g. (0.5 mmoles) of 4-dimethylaminopyridine. The mixture was cooled to 0°C. and saturated with chlorine. The resulting mixture was stirred for 20 hours at room temperature. A sample of the reaction mixture, analyzed by thin-layer chromatography (TLC), indicated that the reaction was not yet complete. The mixture was allowed to stir for an additional 24 hours, and the resulting clear yellow solution was evaporated to obtain crystalline N-chlorophthalimide.

The following Examples illustrate the invention, Examples 1 to 15 illustrating the chlorination process of the invention, Examples 16 and 17 illustrating the production of the novel compound N,N'-dichlorobicyclo[2.2.2]oct-7-ene-2,3,5,6-tetracarboxylic-2,3,5,6-diimide, by processes alternative to that set out in Example 14.

The product was vacuum dried to afford 7.7 g. (84.8%) of product. Analysis: (1) Percent $Cl^+$: Theory: 19.6; Found: 18.3. (2) Percent total chlorine: 18.1. Melting point 183—189°C.

Example 2

To 150 ml. of methylene chloride were added 7.35 g. (0.05 mole) of phthalimide and 17.5 ml. (5 equiv.) of propylene oxide. The resulting mixture was cooled to 0°C., 0.13 g. (0.001 mole; 0.02 equiv.) of quinoline was added, and the mixture was saturated with chlorine. The phthalimide began to dissolve within 30 minutes, and after one hour it was nearly all dissolved. Analysis of the sample by TLC at 1.25 hours showed only a trace of starting material. The mixture was subjected to partial vacuum to remove excess chlorine, and a small amount of amylene was added. The resulting mixture then was evaporated to obtain crystalline N-chlorophthalimide. The product was filtered and vacuum dried overnight to give 8.2 g. (90.4%) of product, essentially identical to the product of Example 1. Analysis: (1) Percent $Cl^+$: Theory: 19.6; Found: 19.3. (2) Percent total chlorine: 19.1.

Example 3

Employing the procedure and scale of Example 2, the same reaction was carried out using 0.08 g. (1 mmole; 0.02 equiv.) of pyridine as catalyst. The reaction was virtually complete after about 1.5 hours and afforded 8.0 g. (88.2%) of N-chlorophthalimide, essentially identical to the product of Example 1. Analysis: (1) Percent $Cl^+$: Theory: 19.6; Found: 19.2. (2) Percent total chlorine: 19.0.

Example 4

Employing the procedure and scale of Example 2, the reaction was carried out using 0.12 g. (1 mmole; 0.02 equiv.) of 2,4,6-trimethylpyridine. The reaction was complete after about two hours, and 8.1 g. (89.3%) of N-chlorophthalimide, essentially identical to the product of Example 1, were recovered. Analysis: (1) Percent $Cl^+$: Theory: 19.5; Found: 18.0. (2) Percent total chlorine: 17.2.

Example 5

Employing the procedure and scale of Example 2, the reaction was carried out using 0.08 g. (1 mmole; 0.02 equiv.) of pyrimidine. The reaction was complete after two hours, and 8.5 g. (93.7%) of N-chlorophthalimide, essentially identical to the product of Example 1, was recovered. Analysis: (1) Percent $Cl^+$: Theory: 19.5; Found: 19.3. (2) Percent total chlorine: 19.0.

Example 6

To 300 ml. of dry methylene chloride were added 9.9 g. (0.1 mole) of succinimide. To the resulting slurry were added 35 ml. (5 equiv.) of propylene oxide and 0.12 g. (1 mmole) of 4-dimethylaminopyridine. The mixture was cooled to 0°C. and saturated with chlorine. The mixture then was stirred for about 18 hours during which it was allowed to warm to room temperature. A sample of the reaction mixture analyzed by TLC indicated little or no starting material. The solvent was slowly evaporated, and the residue was chilled to obtain the crystalline N-chlorosuccinimide product. The product was collected by filtration, washed with a small amount of toluene, and vacuum dried to give 10.6 g. (79.4%) of product as a first crop. Analysis: (1) Percent $Cl^+$: Theory: 26.6; Found: 26.3. (2) Percent total chlorine: 25.9. A second crop of 1.25 g. (9.4%) was recovered. Analysis: (1) Percent $Cl^+$: Theory: 26.6; Found: 25.4. (2) Percent total chlorine: 25.1. Melting point 144—147°C.

Example 7

To 150 ml. of methylene chloride were added 9.15 g. (0.05 mole) of saccharin followed by 17.5 ml. (5 equiv.) of propylene oxide and 0.061 g. (0.5 mmole) of 4-dimethylaminopyridine. The mixture was saturated with chlorine and allowed to stir for 4 days. The solvent then was evaporated, and water was added to the residue. The mixture was filtered, and the collected solid was washed with water and vacuum dried to give 9.2 g. (84.6%) of N-chlorosaccharin. Analysis: (1) Percent $Cl^+$: Theory: 16.3; Found: 14.5. (2) Percent total chlorine: 10.2. Melting point 132—135°C.

Example 8

To 150 ml. of dry methylene chloride were added 7.35 g. (50 mmoles) of phthalimide and 17.5 ml. (250 mmoles) of propylene oxide. The mixture was cooled to 0°C., and 0.11 g. (0.5 mmole) of 1,8-bis(dimethylamino)naphthalene was added. An excess of chlorine was added, and the mixture turned bright orange. The mixture was stirred overnight (about 16 hours) at room temperature. The solvent was evaporated *in vacuo*. Water was added to the resulting crystalline residue, the mixture was filtered, and the collected solid was washed with large volumes of water to give 8.9 g. (98.1%) of N-chlorophthalimide, essentially identical to the product of Example 1. Analysis: (1) Percent $Cl^+$: Theory: 19.5; Found: 19.1. (2) Percent total chlorine: 18.6.

Example 9

Employing the procedure and quantities of Example 8, the reaction was carried out using 0.08 g. (0.5 mmole) of 1,5-diazabicyclo[5.4.0]undec-5-ene (DBU) in place of 1,8-bis-(dimethylamino)naphthalene. N-Chlorophthalimide (8.42 g.; 92.8%), essentially identical to the product of Example 1, was obtained. Analysis: (1) Percent $Cl^+$: Theory: 19.5; Found: 17.4. (2) Percent total chlorine: 16.9.

Example 10

To 150 ml. of methylene chloride were added 7.35 g. (0.05 mole) of phthalimide and 1 drop of DBU. One equiv. of 1,2-epoxy-3-phenoxypropane then was added. The mixture was allowed to react with formation of N-chlorophthalimide.

Example 11

Employing the quantities and conditions of Example 9, the reaction was carried out using 0.07 g. (0.5 mmoles) of N,N-dimethylbenzylamine instead of DBU to obtain 8.5 g. (93.7%) of N-chlorophthalimide, essentially identical to the product of Example 1. Analysis: (1) Percent $Cl^+$: Theory: 19.5; Found: 16.3. (2) Percent total chlorine: 16.7.

Example 12

To 1700 ml. of methylene chloride were added 88.2 g. (0.6 mole) of phthalimide, 210 ml. (5 equiv.) of propylene oxide, and 0.75 g. (6 mmole) of 4-dimethylaminopyridine. The mixture was cooled to 0°C., and an excess of chlorine was added. The mixture was stirred for a total of about 18 hours, and 5 ml. of amylene were then added. The mixture was evaporated *in vacuo* to a volume of about 125 ml. with crystallization of the product. The mixture was chilled in ice for about 1.5 hours and then was filtered. The collected product was washed with a small amount of toluene and was vacuum dried overnight to obtain 103.6 g. (95.1%) of N-chlorophthalimide, essentially identical to the product of Example 1. Analysis: (1) Percent $Cl^+$: Theory: 19.5; Found: 19.1. (2) Percent total chlorine: 19.5.

Example 13

To 150 ml. of dry methylene chloride were added 7.35 g. (0.05 mole) of phthalimide followed by 17.5 ml. (5 equiv.) of propylene oxide and 0.065 g. (0.5 mmoles) of diisopropylethylamine. The mixture was cooled to 0°C. and saturated with chlorine. The mixture was stirred for about 24 hours to provide approximately 50% conversion to N-chlorophthalimide.

Preparation 1

To 1200 ml. of formamide were added 1007.5 g. (4.06 moles) of bicyclo[2.2.2]oct-7-ene-2,3,5,6-tetracarboxylic-2,3,5,6-dianhydride. The mixture was heated, and ammonia addition was begun. Ammonia was added as rapidly as the mixture would accept it. An exothermic reaction occurred. The initial temperature of the mixture was 40°C., and, after 7 minutes, the temperature had risen to 140°C. The product began to crystallize. The ammonia addition was discontinued, and distillation of the solvent was begun. After about 1.75 hours, the temperature of the mixture was about 178°C. Afte 3.75 hours, the temperature of the mixture was 180°C., and 130 ml. of distillate had been collected. Heating was discontinued, and the reaction mixture was allowed to cool under vacuum distillation. After 30 minutes, the temperature of the mixture was 90°C. Vacuum distillation was discontinued, and the reaction mixture was diluted with about 2,000 ml. of acetone. The mixture was cooled in an ice bath to about 5°C. The mixture then was filtered, and the collected product was washed with acetone and dried to give 907.9 g. (90.8%) of bicyclo[2.2.2]oct-7-ene-2,3,5,6-tetracarboxylic-2,3,5,6-diimide.

Example 14

To 1,000 ml. of methylene chloride were added 246.2 g. (1 mole) of bicyclo[2.2.2]oct-7-ene-2,3,5,6-tetracarboxylic-2,3,5,6-diimide, 600 ml. of propylene oxide, and 4.7 ml. (0.04 mole) of quinoline. The mixture was cooled to 20°C., and chlorine addition was begun at a rate sufficient to permit maintenance of the temperature of the mixture at 20—30°C. with ice cooling. After about 40 minutes, the mixture began to thicken, and the chlorine addition rate was reduced. After 2.5 hours the chlorine rate was reduced to a slow stream, and the mixture was stirred gently overnight at about 25—30°C. In the morning, the reaction mixture (25°C.) was white. The rate of chlorine addition was increased, and, after 2.5 hours, the temperature had increased to 30°C. Chlorine addition was discontinued, and the reaction mixture was concentrated *in vacuo* to remove excess propylene oxide and chlorine. After about 25 minutes, the temperature had dropped to 5°C. The reaction mixture was filtered rapidly, and the filter cake was washed successively with 500—1000 ml. of methylene chloride, ether, toluene, and pentane. The filter cake then was air-dried for several hours, and the solid was further dried *in vacuo* at 40—50°C. overnight to obtain 308.4g. of N,N'-dichloro-bicyclo[2.2.2]oct-7-ene-2,3,5,6-tetracarboxylic-2,3,5,6-diimide, m.p. 266°C. (dec.). Analysis: Percent $Cl^+$: Theory: 22.5; Found: 21.7. Some methylene chloride may be retained in the product. This is removed by refluxing the N-chloroimide in toluene.

Example 15

To a 1 l. flask were added 125 g. (from a total of 267.7 g.) of phthalimide, 170.6 g. of cold 1,3-epoxybutane, and 2.3 g. of quinoline. The mixture was cooled to 13°C., stirred, and chlorine was added by means of a tube extending beneath the surface of the mixture. The mixture was maintained at

13—15°C., and about 56 g. of chlorine were added over about 1.5 hours. During the chlorine addition, the mixture first became thicker and then progressively thinner.

The chlorine addition was discontinued, and 96 g. of phthalimide were added to the mixture. The temperature was maintained at 14—15°C., and chlorine addition again was begun. About 40 g. of the chlorine were added over a period of about 0.75 hours during which time the thick mixture again became thinner.

Chlorine addition was discontinued, and the remainder of the phthalimide, providing a total of 267.7 g., was added to the mixture. Chlorine addition again was begun, and about 30 g. of chlorine were added over a period of about 0.5 hours, the temperature again being maintained at 12—15°C.

The mixture was allowed to warm to 21°C. over 0.5 hours. A sample of the mixture was removed and analyzed by thin-layer chromatography (TLC). The analysis indicated the presence of some unreacted starting material. The mixture then was cooled to about 12°C., and 6.5 g. of chlorine were added. The mixture again was allowed to warm to room temperature and filtered to obtain 322.4 g. of N-chlorophthalimide, essentially identical to the product of Example 1. Analysis: Percent $Cl^+$: Theory: 19.5; Found: 18.7.

Example 16

A 369 g. portion of bicyclo[2.2.2]oct-7-ene-2,3,5,6-tetracarboxylic-2,3,5,6-diimide was combined with 369 g. of sodium acetate in 1500 ml. of acetic acid with stirring under a chlorine atmosphere. The temperature was maintained at 40°C. with slight cooling for 1 hour. The temperature was then lowered to 32°C., and the sides of the flask were washed down with a little additional acetic acid. The reaction mixture was then heated to 60°C., and was stirred at that temperature while it was kept saturated with chlorine by bubbling gas through the mixture. The heat and chlorine flow were then turned off, and the mixture was stirred gently for 2 hours more. At the end of this time, its temperature was 27°C. The flask was then washed down with 300 ml. of methylene chloride, and the mixture was cooled in an ice bath. Over a period of 25 minutes, 2500 ml. of ice cold water was added to the mixture. The temperature was then 5°C. The mixture was then filtered, and the solids were washed with 1500 ml. of water and 1500 ml. of methylene chloride. The product was then dried in a vacuum desiccator at 30—40°C. to obtain 472 g. of N,N'-dichlorobicyclo[2.2.2]oct-7-ene-2,3,5,6-tetracarboxylic-2,3,5,6-diimide, substantially identical to the product of Example 14.

Example 17

A portion of bicyclo[2.2.2]oct-7-ene-2,3,5,6-tetracarboxylic-2,3,5,6-diimide is dissolved in methylene chloride. A portion of tertiary butyl hypochlorite is added, and the reaction mixture stirred for 16 hours. The reaction mixture is then evaporated to dryness under vacuum, to obtain crude N,N'-dichlorobicyclo[2.2.2]oct-7-ene-2,3,5,6-tetracarboxylic-2,3,5,6-diimide, which is further purified by recrystallization to obtain the essentially pure compound, substantially identical to the product of Example 14.

## Claims

1. A process for preparing an N-chloroimide which comprises contacting the corresponding cyclic imide containing one or more of the moieties

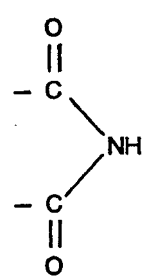

or

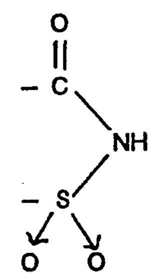

with molecular chlorine characterized in that this contacting is carried out under substantially anhydrous conditions in the presence of (1) an epoxy compound in an amount representing at least about 1 epoxy moiety per imide moiety and (2) at least a catalytic amount of a tertiary amine catalytic transfer agent.

2. A process of claim 1 for preparing an N-chloroimide which comprises contacting with molecular chlorine as described in claim 1, an imide which is chosen from phthalimide, succinimide, saccharin, glutarimide, 3,3-dimethylglutarimide, maleimide, $\Delta^4$-tetrahydrophthalimide, 3,4,5,6-tetrachlorophthalimide, pyromellitic diimide, benzophenone tetracarboxylic diimide, naphthalene 1,4,5,8-tetracarboxylic diimide, chlorendic imide, carbonyl salicylamide, diphenimide, tetrahydrofuran tetracarboxylic imide, 1,8-naphthalimide, and bicyclo[2.2.2]oct-7-ene-2,3,5,6-tetracarboxylic-2,3,5,6-diimide.

3. A process of any of claims 1—2 wherein the temperature is from about −10°C. to about 50°C.

4. A process of claim 3 wherein the epoxy compound is chosen from ethylene oxide, propylene oxide, 1,2-epoxybutane, butadiene diepoxide, 1,2-epoxy-3-phenoxypropane, 1,4-butanediol diglycidyl ether and 1,2,7,8-diepoxyoctane.

5. A process of any of claims 1—4 wherein the tertiary amine is chosen from 1,5-diazabicyclo[4.3.0]non-5-ene, 1,5-diazabicyclo[5.4.0]undec-5-ene, pyridine, 1,8-bis(dimethylamino)naphthalene, 4-dimethylaminopyridine, N,N-dimethylbenzylamine, diisopropylethylamine, quinoline, 2,4,6-trimethylpyridine, pyrimidine and pyrazine.

6. A process of claim 5 wherein the tertiary amine is pyridine, 4-dimethylaminopyridine or 2,4,6-trimethylpyridine.

7. A process of any of claims 1—6 wherein the reaction mixture contains no more than 0.2% water by weight.

8. A process of any of claims 1—7 for preparing N-chlorophthalimide which comprises contacting phthalimide with molecular chlorine under conditions as described in any of claims 1 to 7.

9. A process of any of claims 1—7 for preparing N,N'-dichlorobicyclo[2.2.2]oct-7-ene-2,3,5,6-tetracarboxylic-2,3,5,6-diimide which comprises contacting bicyclo[2.2.2]oct-7-ene-2,3,5,6-tetracarboxylic-2,3,5,6-diimide with molecular chlorine under conditions as described in any of claims 1 to 7.

10. A process of claim 2 for preparing N,N'-dichlorobicyclo[2.2.2]oct-7-ene-2,3,5,6-tetracarboxylic-2,3,5,6-diimide which comprises contacting bicyclo[2.2.2]oct-7-ene-2,3,5,6-tetracarboxylic-2,3,5,6-diimide with molecular chlorine in the presence of propylene oxide and quinoline.

11. N,N'-dichlorobicyclo[2.2.2]oct-7-ene-2,3,5,6-tetracarboxylic-2,3,5,6-diimide.

12. The use of N,N'-dichlorobicyclo[2.2.2]oct-7-ene-2,3,5,6-tetracarboxylic-2,3,5,6-diimide as a chemical reagent which is a source of positive chlorine.

**Patentansprüche**

1. Verfahren zur Herstellung eines N-Chlorimids durch Behandlung des entsprechenden cyclischen Imids, das einen oder mehrere Gruppen der Formeln

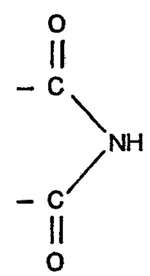

oder

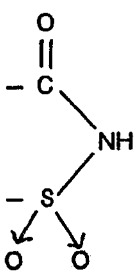

enthält, mit molekularem Chlor, dadurch gekennzeichnet, daß man diese Behandlung unter praktisch wasserfreien Bedingungen in Gegenwart von

(1) einer Epoxyverbindung in einer Menge von wenigstens etwa einem Epoxyrest pro Imidrest und

(2) wenigstens einer katalytischen Menge eines tertiären Amins als katalytischem Übertragungsmittel

durchführt.

2. Verfahren nach Anspruch 1, dadurch gekennzeichnet, daß man als tertiäres Imid (I) Phthalimid, Succinimid, Saccharin, Glutarimid, 3,3-Dimethylglutarimid, Maleimid, $\Delta^4$-Tetrahydrophthalimid, 3,4,5,6-Tetrachlorphthalimid, Pyromellitsäurediimid, Benzophenontetracarbonsä diimid, Naphthalin-1,4,5,8-tetracarbonsäurediimid, Chlorendicsäureimid, Carbonylsalicylimis, Diphenimid, Tetrahydrofurantetracarbonsäureimid, 1,8-Naphthalimid oder Bicyclo[2.2.2]oct-7-en-2,3,5,6-tetracarbonsäure-2,3,5,6-diimid verwendet.

3. Verfahren nach einem der Ansprüche 1 bis 2, dadurch gekennzeichnet, daß man bei einer Temperatur von etwa −10°C bis etwa 50°C arbeitet.

4. Verfahren nach Anspruch 3, dadurch gekennzeichnet, daß man als Epoxyverbindung (1) Ethylenoxid, Propylenoxid, 1,2-Epoxybutan, Butadiendiepoxid, 1,2-Epoxy-3-phenoxypropan, 1,4-Butandioldiglycidylether oder 1,2,7,8-Diepoxyoctan verwendet.

5. Verfahren nach einem der Ansprüche 1 bis 4, dadurch gekennzeichnet, daß man als tertiäres Amin (2) 1,5-Diazabicyclo[4.3.0]non-5-en, 1,5-Diazabicyclo[5.4.0]undec-5-en, Pyridin, 1,8-Bis(dimethylamino)naphthalin, 4-Dimethylaminopyridin, N,N-Dimethylbenzylamin, Diisopropylethylamin, Chinolin, 2,4,6-Trimethylpyridin, Pyrimidin oder Pyrazin verwendet.

6. Verfahren nach Anspruch 5, dadurch gekennzeichnet, daß man als tertiäres Amin (2) Pyridin, 4-Dimethylaminopyridin oder 2,4,6-Trimethylpyridin verwendet.

7. Verfahren nach einem der Ansprüche 1 bis 6, dadurch gekennzeichnet, daß man mit einem Reaktionsgemisch arbeitet, welches nicht mehr als 0,2 Gewichtsprozent Wasser enthält.

8. Verfahren nach einem der Ansprüche 1 bis 7, dadurch gekennzeichnet, daß man Phthalimid mit molekularem Chlor unter den in einem der Ansprüche 1 bis 7 beschriebenen Bedingungen behandelt.

9. Verfahren nach Anspruch 1 bis 7 zur Herstellung von N,N'-Dichlorbicyclo[2.2.2]oct-7-en-2,3,5,6-tetracarbonsäure-2,3,5,6-diimid, dadurch gekennzeichnet, daß man Bicyclo[2.2.2]oct-7-en-2,3,5,6-tetracarbonsäure-2,3,5,6-diimid mit molekularem Chlor unter den in einem der Ansprüche 1 bis 7 beschriebenen Bedingungen behandelt.

10. Verfahren nach Anspruch 2 zur Herstellung von N,N'-Dichlorbicyclo[2.2.2]oct-7-en-2,3,5,6-tetracarbonsäure-2,3,5,6-diimid, dadurch gekennzeichnet, daß man Bicyclo[2.2.2]oct-7-en-2,3,5,6-tetracarbonsäure-2,3,5,6-diimid mit molekularem Chlor in Gegenwart von Propylenoxid und Chinolin behandelt.

11. N,N'-Dichlorbicyclo[2.2.2]oct-7-en-2,3,5,6-tetracarbonsäure-2,3,5,6-diimid.

12. Verwendung von N,N'-Dichlorbicyclo[2.2.2]oct-7-en-2,3,5,6-tetracarbonsäure-2,3,5,6-diimid als chemisches Reagens, welches als Quelle für positives Chlor dient.

**Revendications**

1. Procédé de préparation d'un N-chloroimide comprenant la mise en contact de l'imide cyclique correspondant contenant une ou plusieurs des fractions

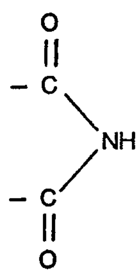

ou

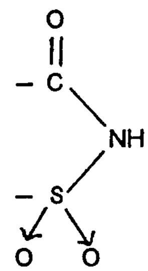

avec du chlore moléculaire, caractérisé en ce que cette mise en contact s'effectue dans conditions pratiquement anhydres en présence de (1) composé époxy en une quantité représentant au moins environ 1 fraction époxy par fraction imide et (2) au moins une quantité catalytique d'un agent de transfert catalytique amine tertiaire.

2. Procédé selon la revendication 1 de préparation d'un N-chloroimide qui comprend la mise en contact avec du chlore moléculaire comme il est dit dans la revendication 1, d'un imide choisi parmi le phtalimide, le succinimide, la saccharine, le glutarimide, le 3,3-diméthylglutarimide, le maléimide, le $\Delta^4$-tétrahydrophtalimide, le 3,4,5,6-tétrachlorophtalimide, le diimide pyromellitique, le benzophenone-diimide tétracarboxylique, le naphtalène-diimide 1,4,5,8-tétracarboxylique, l'imide chlorendique, le carbonylsalicylamide, le diphénimide, le tétreahydrofuranneimide tétracarboxylique, le 1,8-naphthalimide et le bicyclo(2,2,2)oct-7-ène-2,3,5,6-tétracarboxylique-2,3,5,6-diimide.

3. Procédé selon l'une quelconque des revendications 1 et 2 où le température varie d'environ −10°C à environ 50°C.

4. Procédé selon la revendication 3 où le composé époxy est choisi parmi l'éthylène-oxyde, le propylène-oxyde, le 1,2-époxybutane, le butadiènediépoxide, le 1,2-époxy-3-phénoxypropane, le 1,4-butanediol-diglycidyl-éther et le 1,2,7,8-diépoxyoctane.

5. Procédé selon l'une quelconque des revendications 1 à 4, où l'amine tertiaire est choisie parmi le 1,5-diazabicyclo[4,3,0]non-5-ène, le 1,5-diazabicyclo[5,4,0]undéc-5-ène, la pyridine, le 1,8-bis(diméthylamino)naphtalène, la 4-diméthyl-aminopyridine, la N,N-diméthylbenzylamine, la diisopropyléthylamine, la quinoléine, la 2,4,6-triméthylpyridine, la pyrimidine et la pyrazine.

6. Procédé selon la revendication 5 où l'amine tertiaire est la pyridine, le 4-diméthylamino-pyridine ou la 2,4,6-triméthylpyridine.

7. Procédé selon l'une quelconque des revendications 1 à 6 où le mélange réactionnel ne contient pas plus de 0,2% en poids d'eau.

8. Procédé selon l'une qualconque des revendications 1 à 7 de préparation de N-chlorophtalimide qui comprend la mise en contact du phtalimide avec du chlore moléculaire dans des conditions telles que décrites dans l'une quelconque des revendications 1 à 7.

9. Procédé selon l'une quelconque des revendications 1 à 7 de préparation du N,N'-dichloro-bicyclo[2,2,2]oct-7-ène-2,3,5,6-tétracarboxylique-2,3,5,6-diimide qui comprend la mise en contact du bicyclo[2,2,2]oct-7-ène-2,3,5,6-tétracarboxylique-2,3,5,6-diimide avec du chlore moléculaire dans des conditions telles que décrites dans l'une quelconque des revendications 1 à 7.

10. Procédé selon la revendication 2 de préparation du N,N'-dichlorobicyclo[2,2,2]oct-7-ène-2,3,5,6-tétracarboxylique-2,3,5,6-diimide qui comprend la mise en contact du bicyclo[2,2,2]oct-7-ène-2,3,5,6-tétracarboxylique-2,3,5,6-diimide avec du chlore moléculaire en présence de propylènoxyde et de quinoléine.

11. N,N'-dichlorobicyclo[2,2,2]oct-7-ène-2,3,5,6-tétracarboxylique-2,3,5,6-diimide.

12. Application du N,N'-dichlorobicyclo[2,2,2]oct-7-ène-2,3,5,6-tétracarboxylique-2,3,5,6-diimide comme réactif chimique qui est une source de chlore positif.